# EUROPEAN PATENT SPECIFICATION

(11) **EP 1 737 381 B1**
(45) Date of publication and mention of the grant of the patent: **29.09.2010**
(21) Application number: 05737027.2
(22) Date of filing: 18.04.2005
(51) Int. Cl.: A61F 2/01

(54) **REMOVABLE VENA CAVA FILTER**
ENTFERNBARER VENA CAVA FILTER
FILTRE RETIRABLE POUR LA VEINE CAVE

(30) Priority: 16.04.2004 US 563243 P; 16.04.2004 US 562813 P; 16.04.2004 US 562909 P; 16.04.2004 US 563176 P
(43) Date of publication of application: 03.01.2007
(73) Proprietor: Cook Incorporated, Bloomington, IN 47402-0489 (US); Wilson Cook Europe ApS, 4632 Bjaeverskov (DK)
(72) Inventor: OSBORNE, Thomas, A., Bloomington, IN 47401 (US); MOLGAARD-NIELSEN, Arne, DK-2100 Oesterbro (DK); HENDRIKSEN, Per, DK-4850 Stubbekoebing (DK); GUNTHER, Rolf, 52057 Aachen (DE)
(74) Representative: Garratt, Peter Douglas
(86) International application number: PCT/US2005/013160
(87) International publication number: WO 2005/102211

(56) References cited:
- EP-A- 0 350 043
- US-A- 5 836 968
- US-A- 6 126 673
- US-A1- 2004 193 209
- US-A1- 2004 230 220

## Description

### CROSS-REFERENCE TO RELATED APPLICATIONS

This application claims the benefit of U.S. Provisional Application serial no. 60/563,243, filed on April 16, 2004, entitled "REMOVABLE VENA CAVA FILTER".

This application also claims the benefit of U.S. Provisional Application no. 60/562,813, filed on April 16, 2004, entitled "REMOVABLE FILTER FOR CAPTURING BLOOD CLOTS".

This application also claims the benefit of U.S. Provisional Application no. 60/562,909, filed on April 16, 2004, entitled "BLOOD CLOT FILTER WITH STRUTS HAVING AN EXPANDED REMEMBERED STATE,".

This application also claims the benefit of U.S. Provisional Application no. 60/563,176, filed on April 16, 2004, entitled "BLOOD CLOT FILTER HAVING A COLLAPSED REMEMBERED STATE".

### BACKGROUND OF THE INVENTION

The present invention relates to medical devices. More particularly, the invention relates to a removable vena cava clot filter that can be percutaneously placed in and removed from the vena cava of a patient.

Filtering devices that are percutaneously placed in the vena cava have been available for over thirty years. A need for filtering devices arises in trauma patients, orthopedic surgery patients, neurosurgery patients, or in patients having medical conditions requiring bed rest or non-movement. During such medical conditions, the need for filtering devices arises due to the likelihood of thrombosis in the peripheral vasculature of patients wherein thrombi break away from the vessel wall, risking downstream embolism or embolization. For example, depending on the size, such thrombi pose a serious risk of pulmonary embolism wherein blood clots migrate from the peripheral vasculature through the heart and into the lungs.

A filtering device can be deployed in the vena cava of a patient when, for example, anticoagulant therapy is contraindicated or has failed. Typically, filtering devices are permanent implants, each of which remains implanted in the patient for life, even though the condition or medical problem that required the device has passed. In more recent years, filters have been used or considered in preoperative patients and in patients predisposed to thrombosis which places the patient at risk for pulmonary embolism.

The benefits of a vena cava filter have been well established, but improvements may be made. For example, filters generally have not been considered removable from a patient due to the likelihood of endotheliosis of the filter or fibrous reaction matter adherent to the endothelium during treatment. After deployment of a filter in a patient, proliferating intimal cells begin to accumulate around the filter struts which contact the wall of the vessel. After a length of time, such ingrowth prevents removal of the filter without risk of trauma, requiring the filter to remain in the patient. As a result, there has been a need for an effective filter that can be removed after the underlying medical condition has passed.

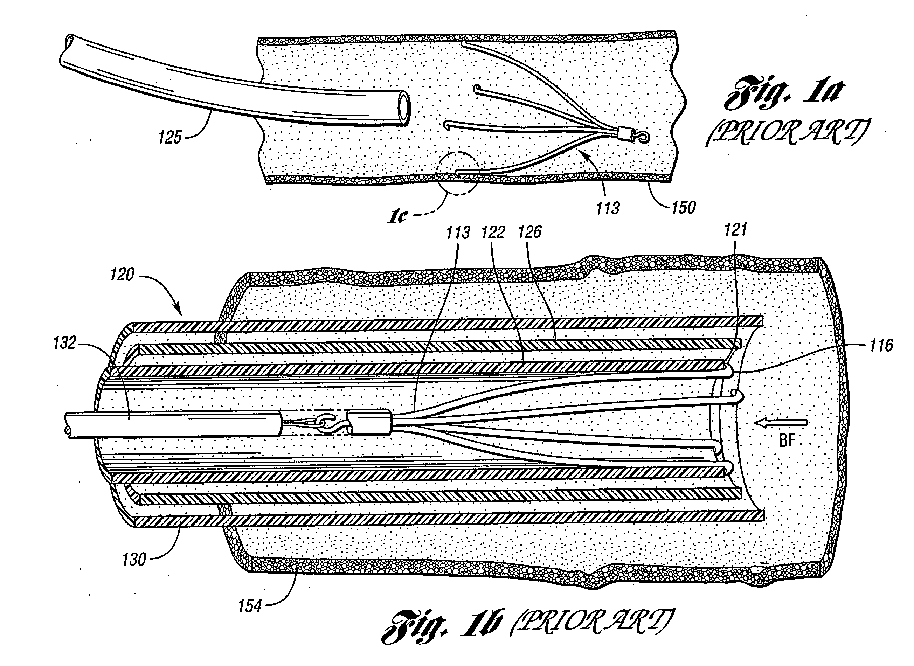

Conventional filters commonly become off-centered or tilted with respect to the hub of the filter and the longitudinal axis of the vessel in which it has been inserted. As a result, the filter including the hub and the retrieval hook engage the vessel wall along their lengths and potentially become endothelialized therein. This condition is illustrated in prior art Figure 1a in which a prior art filter 113 has been delivered by a delivery sheath 125 through the vessel 150 of a patient. In the event of this occurrence, there is a greater likelihood of endotheliosis of the filter to the blood vessel along a substantial length of the filter wire. As a result, the filter becomes a permanent implant in a shorter time period than otherwise.

Furthermore, further improvements may be made related to the delivery or retrieval of vena cava filters. For delivery of vena cava filters, an introducer system having an introducer tube may be percutaneously inserted in the vena cava of a patient through the femoral vein or the jugular vein. A part of an introducer assembly 120 is illustrated in prior art Figure 1b in which the prior art filter 113 is percutaneously delivered through the jugular vein 154 of a patient. As shown, the filter 113 in its collapsed configuration is placed at the distal end 121 of an inner sheath 122 with anchoring hooks 116 of the filter 113 extending past the distal end 121. An outer sheath 126 is then disposed over the inner sheath 122 to avoid undesirably scratching or scraping of the anchoring hooks 116 against the introducer tube 130. The inner and outer sheaths 122, 126 along with a pusher member 132 are then moved together through the introducer tube 130 to deliver the filter 113 to the vena cava of the patient.

It has been a challenge to design a vena cava filter with features that lessen the concerns of undesirably scratching or scraping of the anchoring hooks against outer walls of an introducer tube or a blood vessel while maintaining the effectiveness of the filter.
[0011a] Reference is directed to US 6 126 673 wherein a vascular filter comprises an emboli-capturing portion having a set of helical filter-wires joined at a central region and extending in a given direction along the blood vessel in a diverging relationship to the axis of the filter, the wires terminating in free ends constructed to engage the walls of said vessel. A major mid-portion of the length of the free ended wires are of generally helical form, cooperatively related to form an effective emboli capturing array. Anchoring is accomplished by a separate assembly formed of struts and anchoring devices. A parallelogram supporting strut assembly and other members for providing linear engagement with the wall of the vena cava are shown.

### BRIEF SUMMARY OF THE INVENTION

One embodiment of the present invention generally provides a removable vena cava filter configured for simplified delivery to and retrieval from the vena cava of a patient according to claim 1. The filter includes primary and secondary struts extending along a longitudinal axis. Each primary strut has a first thickness and a first tensile strength. Each secondary strut has a second thickness and a second tensile strength, wherein the first thickness is greater than the second thickness and the first tensile strength is greater than the second tensile strength.

The present invention provides a removable vena cava filter for capturing thrombi in a blood vessel. In one embodiment, the filter comprises a plurality of primary struts having first ends attached together along a longitudinal axis. Each primary strut includes an arcuate segment having a first tensile strength. The arcuate segment extends from the first end to an anchoring hook. The anchoring hook is integral with the arcuate segment and having first tensile strength. The filter further comprises a plurality of secondary struts spaced between the primary struts and having connected ends attached together along the longitudinal axis. Each secondary strut extends from the connected end to a free end. Each secondary strut has a second tensile strength.

In another embodiment, each of the primary struts has a first thickness and each of the secondary struts has a second thickness less thick than the first thickness. The removable filter further includes a hub configured to axially house the first ends of the plurality of primary struts and a retrieval hook extending from the hub opposite the plurality of primary struts for removal of the filter from the blood vessel.

In certain embodiments, pairs of secondary struts are positioned between pairs of primary struts. Each pair of secondary struts is twisted together near the connected ends of the secondary struts to form a twisted section. The twisted sections of the secondary struts effectively stiffen the struts to enhance their centering capabilities to prevent the filter from tilting when the filter is deployed in the blood vessel. Hence, engagement between the struts and the blood vessel is minimized which reduces the potential for the struts to become endothelialized within the blood vessel. A further feature of the twisted sections is that they prevent or at least minimize the secondary struts from entangling with the primary struts.

Further aspects, features, and advantages of the invention will become apparent from consideration of the following description and the appended claims when taken in connection with the accompanying drawings.

### BRIEF DESCRIPTION OF THE DRAWINGS

Figure 1 a is a side view of a prior art filter deployed through the vasculature of a patient;

Figure 1b is a side view of an introducer assembly including the prior art filter to be delivered to the vena cava of a patient;

Figure 2 is an illustration of the anatomy of the renal veins, the iliac veins, and the vena cava in which one embodiment of a vena cava filter of the present invention is deployed;

Figure 3a is a side perspective view of one embodiment of the vena cava filter in an expanded state;

Figure 3b is a side view of the vena cava filter of Figure 3a in a collapsed state and disposed in an introducer tube;

Figure 4 is an enlarged view of a portion of a second arcuate portion of a primary strut of the vena cava filter;

Figure 5 is a cross-sectional view of a hub of the filter in Figure 3 taken along line 5-5;

Figure 6a is a cross-sectional view of the vena cava depicting the filter partially deployed leading with the removal hook;

Figure 6b is a cross-sectional view of the vena cava depicting the filter partially deployed leading with the anchoring hooks;

Figure 7 is a cross-sectional view of the vena cava in which the filter of Figure 3 has been deployed;

Figure 8a is a cross-sectional view of the vena cava of Figure 7a taken along line 8-8;

Figure 8b is a cross-sectional view of the vena cava of Figure 7a taken along line 8-8 depicting another embodiment of the filter;

Figure 9a is a cross-sectional view of a blood vessel in which a retrieval sheath engages primary struts of the filter in Figure 3 for removal;

Figure 9b is a cross-sectional view of a blood vessel in which the retrieval sheath includes the filter in the collapsed state for removal;

Figure 10 is a cross-sectional view of a blood vessel showing a vena cava filter deployed within the blood vessel in accordance with another embodiment of the invention; and

Figure 11 is a view of the blood vessel and filter of Figure 10 taken along the line 11-11.

### DETAILED DESCRIPTION OF THE INVENTION

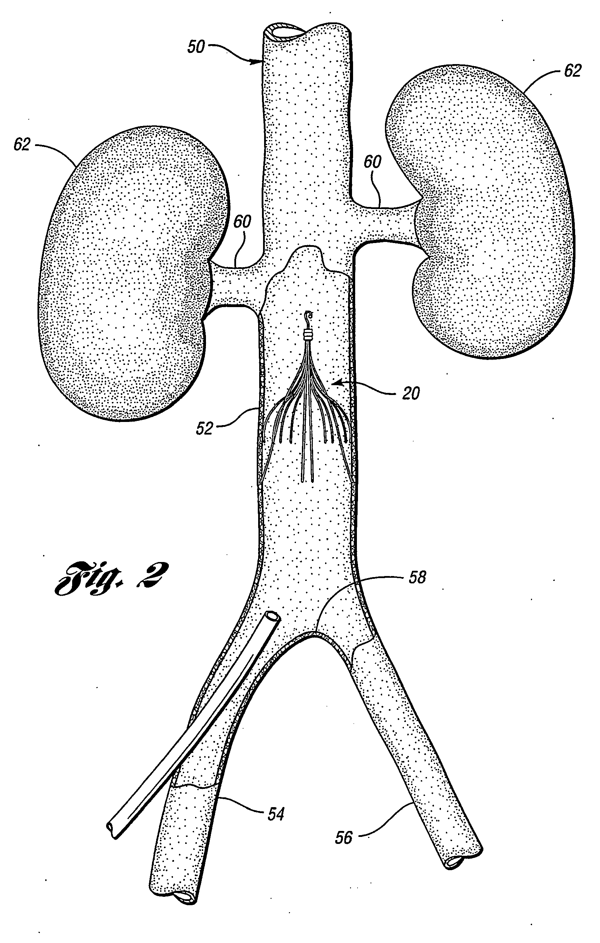

In accordance with one embodiment of the present invention, Figure 2 illustrates a vena cava filter 10 implanted in the vena cava 50 for the purpose of lysing or capturing thrombi carried by the blood flowing through the iliac veins 54, 56 toward the heart and into the pulmonary arteries. As shown, the iliac veins merge at juncture 58 into the vena cava 50. The renal veins 60 from the kidneys 62 join the vena cava 50 downstream of juncture 58. The portion of the vena cava 50, between the juncture 58 and the renal veins 60, defines the inferior vena cava 52 in which the vena cava filter 10 has been percutaneously deployed through one of the iliac veins 54. Preferably, the vena cava filter 10 has a length smaller than the length of the inferior vena cava 52. If the lower part of the filter extends into the iliac veins, filtering effectiveness will be compromised and if the filter wires cross over the origin of the renal veins the filter wires might interfere with the flow of blood from the kidneys.

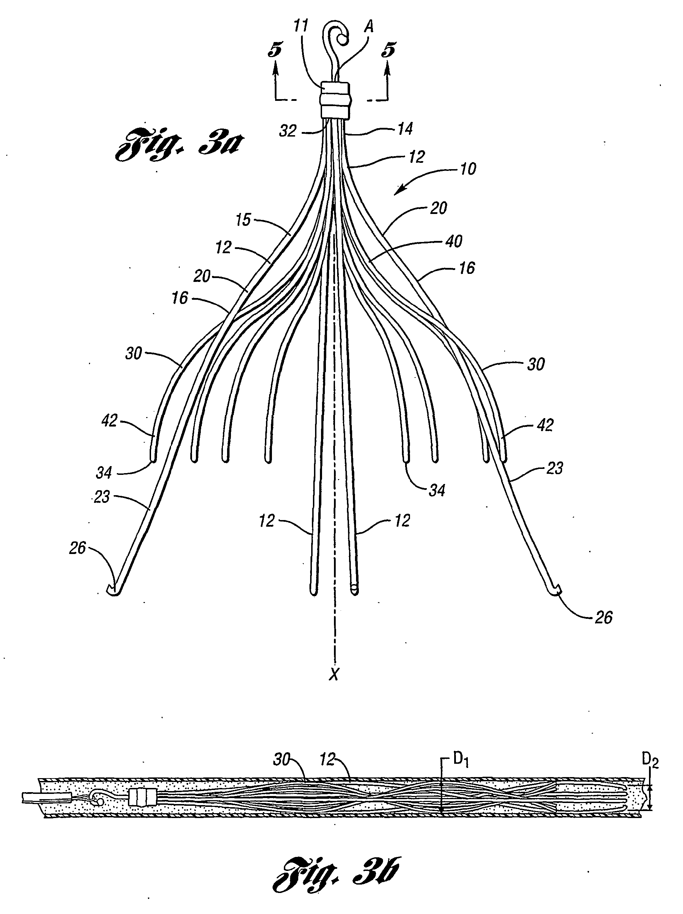

This embodiment of the present invention will be further discussed with reference to Figures 3-9 in which filter 10 is shown. Figure 3a illustrates filter 10 in an expanded state and comprising four primary struts 12 each having first ends that emanate from a hub 11. Hub 11 attaches by crimping first ends 14 of primary struts 12 together at a center point A in a compact bundle along a central or longitudinal axis X of the filter. The hub 11 has a minimal diameter for the size of wire used to form the struts.

Preferably, the primary struts 12 are formed of a superelastic material, stainless steel wire, Nitinol, colbalt-chromium-nickel-molybdenum-iron alloy, cobalt- chrome alloy, or any other suitable superelastic material that will result in a self- opening or self-expanding filter. In this embodiment, the primary struts 12 are preferably formed from wire having a round cross-section with a diameter of at least about 0.038 cm (0.015 inches). Of course, it is not necessary that the primary struts have a round or near round cross-section. For example, the primary struts 12 could take on any shape with rounded edges to maintain non-turbulent blood flow therethrough.

Each primary strut 12 includes an arcuate segment 16 having a soft S- shape. Each arcuate segment 16 is formed with a first curved portion 20 that is configured to softly bend away from the longitudinal or central axis X of the filter 10 and a second curved portion 23 that is configured to softly bend toward the longitudinal axis of the filter 10. Due to the soft bends of each arcuate segment 16, a prominence or a point of inflection on the, primary strut 12 is substantially avoided to aid in non-traumatically engaging the vessel wall.

As shown in Figure 3a, the primary struts 12 terminate at anchoring hooks 26 that will anchor in the vessel wall when the filter 10 is deployed at a delivery location in the blood vessel. The primary struts 12 are configured to move between an expanded state for engaging the anchoring hooks 26 with the blood vessel and a collapsed state for filter retrieval or delivery.

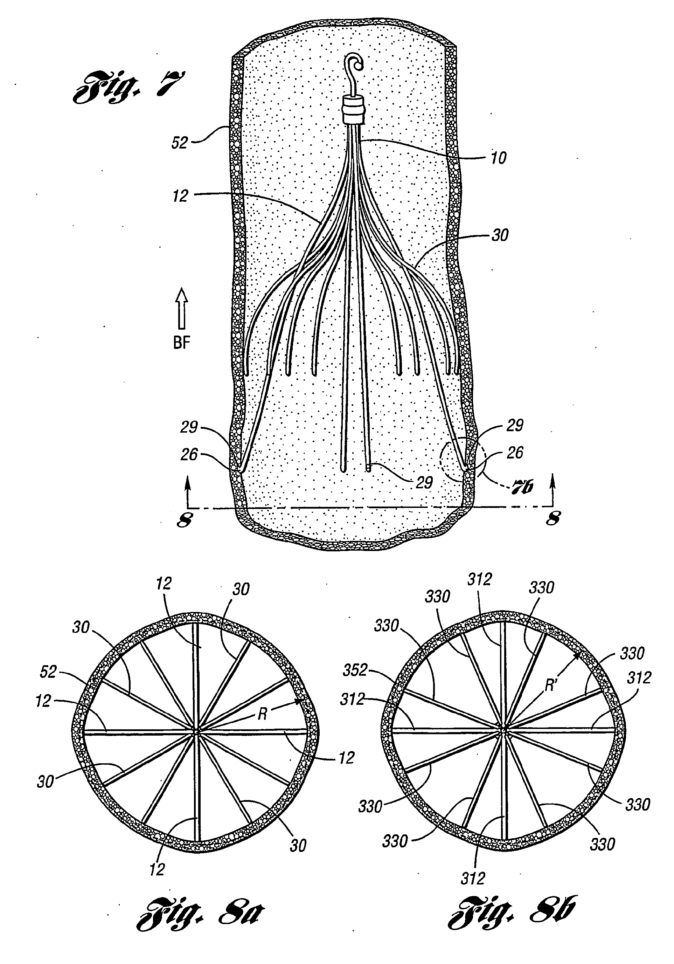

In the expanded state, each arcuate segment 16 extends arcuately along a longitudinal axis (as shown in Figure 3a) and linearly relative to a radial axis (as shown in Figure 8a) from the first end 14 to the anchoring hook 26. The primary struts 12 extend linearly relative to the radial axis and avoid entanglement with other struts. In this embodiment, the filter 10 extends longitudinally as shown in Figure 3a, defining the longitudinal axis X of filter 10. The filter 10 further radially expands and collapses as shown in Figure 8a, defining the radial axis R of the filter 10.

As discussed in greater detail below, the soft bends of each arcuate segment 16 allow each primary strut 12 to cross another primary strut 12 along the longitudinal axis X in the collapsed state such that each anchoring hook 26 faces the longitudinal axis X for filter retrieval or delivery.

When the filter 10 is deployed in a blood vessel, the anchoring hooks 26 engage the walls of the blood vessel to define a first axial portion to secure the filter in the blood vessel. The anchoring hooks 26 prevent the filter 10 from migrating from the delivery location in the blood vessel where it has been deposited. The primary struts 12 are shaped and dimensioned such that, when the filter 10 is freely expanded, the filter 10 has a diameter of between about 25 mm and 45 mm and a length of between about 3 cm and 7 cm. For example, the filter 10 may have a diameter of about 35 mm and a length of about 5 cm. The primary struts 12 have sufficient spring strength that when the filter is deployed the anchoring hooks 26 will anchor into the vessel wall.

As shown in Figure 3a, the filter 10 further includes a plurality of secondary struts 30 having connected ends 32 that independently emanate from hub 11. Hub 11 attaches by crimping the connected ends 32 alt the center point A of the secondary struts 30 together with the primary struts 12. In this embodiment, each primary strut 12 has two secondary struts 30 in side-by-side relationship with the primary strut 12. The secondary struts 30 independently extend from the connected ends 32 to free ends 34 to centralize the filter 10 in the expanded state in the blood vessel. As shown, each secondary strut 30 freely extends from the hub 11, avoiding contact with other struts. This configuration lessens the likelihood of entanglement. As shown, each secondary strut 30 extends arcuately along a longitudinal axis and linearly relative to a radial axis from the connected end 32 to the free end 34 for engaging the anchoring hooks 26 with the blood vessel. As with the primary struts 12, the secondary struts 30 extend linearly relative to the radial axis and avoid entanglement with other struts.

The secondary struts 30 may be made from the same type of material as the primary struts 12. In this embodiment, the secondary struts 30 have a smaller diameter, e.g., at least about 0.030 cm (0.012 inches), than the primary struts 12. In this embodiment, each of the secondary struts 30 is formed of a first arc 40 and a second arc 42. The first arc 40 extends from the connected end 32 away from the longitudinal axis X. The second arc 42 extends from the first arc 40 towards the longitudinal axis X. As shown, two secondary struts 30 are located on each side of one primary strut 12 to form a part of a netting configuration of the filter 10. The hub 11 is preferably made of the same material as the primary struts and secondary struts to minimize the possibility of galvanic corrosion or molecular changes in the material due to welding.

When freely expanded, free ends 34 of the secondary struts 30 will expand radially outwardly to a diameter of about 25 mm to 45 mm to engage the vessel wall. For example, the secondary struts 30 may expand radially outwardly to a diameter of between about 35 mm and 45 mm. The second arcs 42 of the free ends 34 engage the wall of a blood vessel to define a second axial portion where the vessel wall is engaged. The secondary struts 30 function to stabilize the position of the filter 10 about the center of the blood vessel in which it is deployed. As a result, the filter 10 has two layers or portions of struts longitudinally engaging the vessel wall of the blood vessel. The length of the filter 10 is preferably defined by the length of a primary strut 12.

Furthermore, the diameter of the hub 11 is defined by the size of a bundle containing the primary struts 12 and secondary struts 30. In this embodiment, the eight secondary struts 30 minimally add to the diameter of the hub 11 or the overall length of the filter 10, due to the reduced diameter of each secondary strut 30. This is accomplished while maintaining the filter 10 in a centered attitude relative to the vessel wall and formed as a part of the netting configuration of the filter 10. As shown, removal hook 46 extends from hub 11 opposite primary and secondary struts 12 and 30.

In this embodiment, each arcuate segment 16 has an absolute tensile strength of between about 1.965 Mega Pascal (MPa) (285,000 pounds per square inch (psi)) and 2.275 MPa (330,000 psi). Each anchoring hook 26 is integral with the arcuate segment 16 and has the same thickness and absolute tensile strength of the arcuate segment. Each secondary strut 30 has an absolute tensile strength of between about 1.965 MPa (285,000 psi) and 2.275 MPa (330,000 psi). In one embodiment, the absolute tensile strength may be defined as the maximum load of cross-section sustained by a strut.

Figure 3b illustrates the filter 10 in a collapsed state disposed in a delivery/retrieval tube 94 for delivery or retrieval. As shown, the filter 10 is shaped for each primary strut 12 to cross another primary strut 12 along the longitudinal axis X. As a result, in the collapsed state, the anchoring hooks 26 are configured to invert or inwardly face the longitudinal axis X for retrieval and delivery of the filter 10. This inverted or inwardly facing configuration of the anchoring hooks 26 allows for simplified delivery and retrieval of filter 10.

In the collapsed state, each primary strut 12 is configured to cross another primary strut 12 along the longitudinal axis X such that the arcuate segments 16, first curved portions 20 or second curved portions 23, occupy a first diameter Di. In this embodiment, the first diameter is greater than a second diameter D2 occupied by the anchoring hooks 26 for filter retrieval or delivery. It has been found that the first diameter of the arcuate segments 16 serves to clear a path of retrieval, reducing radial force from the sheath or blood vessel on the anchoring hooks 26 during removal of the filter 10 from a patient. Reducing the radial force on the anchoring hooks 26 assists in preventing the anchoring hooks 26 from scraping, scratching, or tearing the inner wall of a sheath during removal of the filter 10 from a patient.

In this embodiment of the present invention, it is to be noted that the filter 10 may be delivered or retrieved by any suitable introducer (delivery or retrieval) tube. However, it is preferred that the introducer tube has an inside diameter of between about 4.5 French and 16 French, and more preferably between about 6.5 French and 14 French.

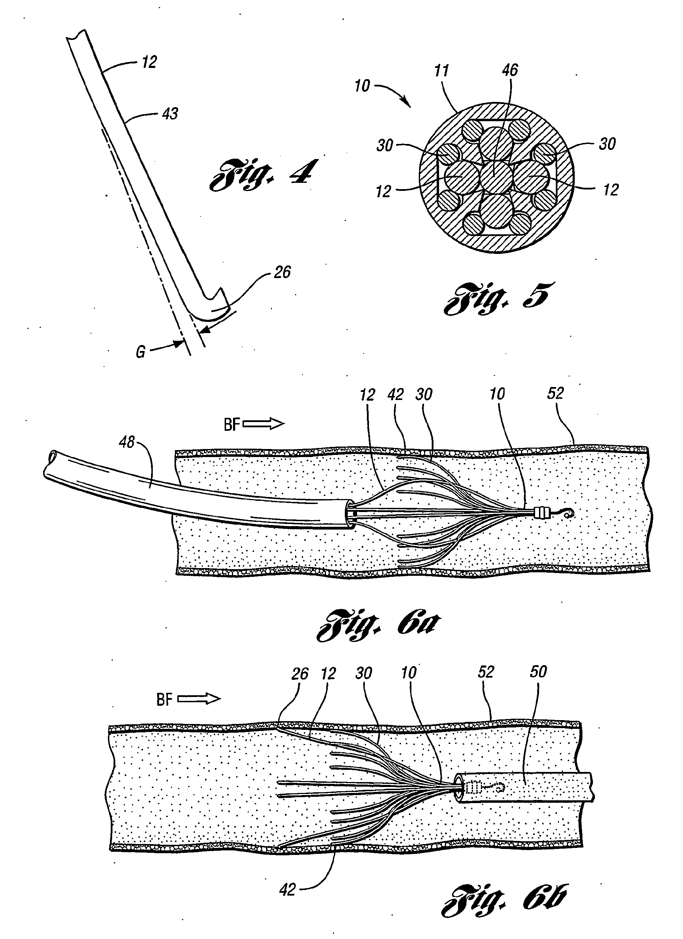

Figure 4 illustrates primary strut 12 including distal bend 43 formed thereon and extending outwardly radially from the longitudinal axis X. As shown in Figure 4, the distal bend 43 may extend outwardly at an angle between about 0.5 degree to 2 degrees, preferably 1.0 degree. The distal bend 43 allows the filter 10 to filter thrombi effectively at a smaller inside diameter of a blood vessel than otherwise would be possible while maintaining the ability to collapse for delivery or retrieval.

Figure 5 illustrates a cross-sectional view of the filter 10 of Figure 3a at hub 11. As shown, the hub 11 houses a bundle of first ends 14 of the four primary struts 14 and connected ends 32 of secondary struts 30. Figure 5 further depicts the configurations of the primary and secondary struts 12 and 30. In this embodiment, the primary struts 12 are spaced between two secondary struts 30. Of course, the primary struts 12 may be spaced between any other suitably desired number of secondary struts 30.

In this embodiment, Figures 6a and 6b both illustrate the filter 10 partially deployed in inferior vena cava 52. Figure 6a shows the filter 10 being delivered by a delivery tube 48 through the femoral vein of a patient and Figure 6b shows the filter 10 being delivered by a delivery tube 50 through the jugular vein of a patient. For deployment of the filter 10, a delivery tube is percutaneously inserted through the patient's vessel such that the distal end of the delivery tube is at the location of deployment. In this embodiment, a wire guide is preferably used to guide the delivery tube to the location of deployment. In Figure 6a, the filter 10 is inserted through the proximal end of the delivery tube 48 with the removal hook 46 leading and anchoring hooks 26 of the primary struts 12 held by a filter retainer member for delivery via the femoral vein of a patient.

. In Figure 6b, the filter 10 is inserted through the proximal end of the delivery tube 50 with the anchoring hooks 26 of the primary struts 12 leading and the removal hook 46 trailing for delivery via the jugular vein of a patient. In this embodiment, a pusher wire having a pusher member at its distal end may be fed through the proximal end of the delivery tube 50 thereby pushing the filter 10 until the filter 10 reaches the distal end of the delivery tube 50 to a desired location.

During deployment, the secondary struts 30 expand first to centralize or balance the filter within the vessel. When the free ends of the secondary struts emerge from the distal end of either of the delivery tubes 48 or 50, the secondary struts 30 expand to an expanded position as shown in both Figures 6a and 6b. The second arcs 42 engage the inner wall of the vessel. The second arcs 42 of the secondary struts 30 function to stabilize the attitude of filter 10 about the center of the blood vessel. When delivering through the jugular vein (Figure 6b), the filter 10 is then pushed further by the pusher wire (not shown) until it is fully deployed.

When the filter 10 is fully expanded in the vena cava, the anchoring hooks 26 of the primary struts 12 and the second arcs 42 of the secondary struts 30 are in engagement with the vessel wall. The anchoring hooks 26 of the primary struts 12 have anchored the filter 10 at the location of deployment in the vessel, preventing the filter 10 from moving with the blood flow through the vessel. As a result, the filter 10 is supported by two sets of struts that are spaced axially along the length of the filter.

Figure 7 illustrates the filter 10 fully expanded after being deployed in inferior vena cava 52. As shown, the inferior vena cava 52 has been broken away so that the filter 10 can be seen. The direction of the blood flow BF is indicated in Figure 7 by the arrow that is labeled BF. The anchoring hooks 26 at the ends of the primary struts 12 are shown as being anchored in the inner lining of the inferior vena cava 52. The anchoring hooks 26 include barbs 29 that, in one embodiment, project toward the hub 11 of the filter. The barbs 29 function to retain the filter 10 in the location of deployment. The spring biased configuration of the primary struts 12 further causes the anchoring hooks 26 to engage the vessel wall and anchor the filter at the location of deployment. After initial deployment, the pressure of the blood flow on the filter 10 contributes in maintaining the barbs 29 anchored in the inner lining of the inferior vena cava 52. As seen in Figure 7, the second arcs 42 of secondary struts 30 also have a spring biased configuration to engage with the vessel wall.

As seen in Figure 7, the hub 11 and removal hook 46 are positioned downstream from the location at which the anchoring hooks 26 are anchored in the vessel. When captured by the struts 12 and 30, thrombi remains lodged in the filter. The filter 10 along with the thrombi may then be percutaneously removed from the vena cava. When the filter 10 is to be removed, the removal hook 46 is preferably grasped by a retrieval instrument that is percutaneously introduced in the vena cava in the direction of removal hook 16 first.

Figure 8a depicts a netting configuration or pattern formed by the primary struts 12, secondary struts 30, and the hub 11 relative to radial axis R. The netting pattern shown in Figure 8a functions to catch thrombi carried in the blood stream prior to reaching the heart and lungs to prevent the possibility of a pulmonary embolism. The netting pattern is sized to catch and stop thrombi that are of a size that are undesirable to be carried in the vasculature of the patient. Due to its compacted size, the hub minimally resists blood flow.

Figure 8a depicts the netting pattern including primary struts and secondary struts at substantially equal angular space relative to each other. The netting pattern provides an even distribution between the primary and secondary struts to the blood flow, increasing the likelihood of capturing thrombi. However, as shown in Figure 8b, it is to be understood that each of the sets of primary struts 312 and secondary struts 330 may be independently spaced substantially equally at their respective portions relative to radial axis R'. For example, the secondary struts 330 may be spaced equally relative to the other secondary struts 330 and the primary struts 312 may be spaced equally relative to the other primary struts 312. As a result, the netting pattern in this embodiment shown by the cross-sectional view of the vena cava (taken along line 8-8) will have uneven or unequal spacing between the primary struts 312 and secondary struts 330.

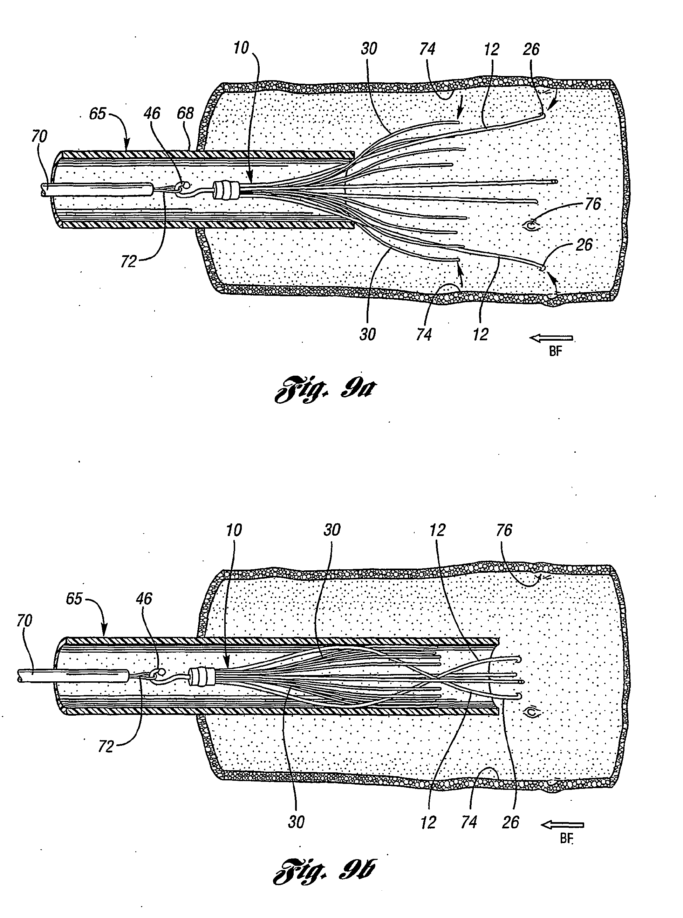

Figures 9a and 9b illustrates part of a retrieval device 65 being used in a procedure for removing the filter 10 from the inferior vena cava 52. The retrieval device 65 is percutaneously introduced into the superior vena cava via the jugular vein. In this procedure, a removal catheter or sheath 68 of the retrieval device 65 is inserted into the superior vena cava. A wire 70 having a loop snare 72 at its distal end is threaded through the removal sheath 68 and is exited through the distal end of the sheath 68. The wire 70 is then manipulated by any suitable means from the proximal end of the retrieval device such that the loop snare 72 captures the removal hook 46 of the filter 10. Using counter traction by pulling the wire 70 while pushing the sheath 68, the sheath 68 is passed over the filter 10. As the sheath 68 passes over the filter 10, the primary struts 12 and then the secondary struts 30 engage the edge of the sheath 68 and are caused to pivot or undergo bend deflection at the hub 11 toward the longitudinal axis of the filter. The pivoting toward the longitudinal axis causes the ends of the struts 12 and 30 to be retracted from the vessel wall. In this way, only surface lesions 74 and small point lesions 76 on the vessel wall are created in the removal procedure. As shown, the surface lesions 74 are created by the ends of the secondary struts 30 and the small point legions 76 are created by the anchoring hooks 26 of the primary struts 12. However, it is to be noted that any other suitable procedure may be implemented to remove the filter from the patient.

The primary and secondary struts can be formed from any suitable material that will result in a self-opening or self-expanding filter, such as shape memory alloy. Shape memory alloys have the desirable property of becoming rigid, that is, returning to a remembered state, when heated above a transition temperature. A shape memory alloy suitable for the present invention is Ni-Ti available under the more commonly known name Nitinol. When this material is heated above the transition temperature, the material undergoes a phase transformation from martensite to austenic, such that material returns to its remembered state. The transition temperature is dependent on the relative proportions of the alloying elements Ni and Ti and the optional inclusion of alloying additives.

In other embodiments, both the primary struts and the secondary struts are made from Nitinol with a transition temperature that is slightly below normal body temperature of humans, which is about 37°C (98.6°F). Thus, when the filter is deployed in the vena cave and exposed to normal body temperature, the alloy of the struts will transform to austenite, that is, the remembered state, which for the present invention is an expanded configuration when the filter is deployed in the blood vessel. To remove the filter, the filter is cooled to transform the material to martensite which is more ductile than austenite, making the struts more malleable. As such, the filter can be more easily collapsed and pulled into the sheath for removal.

In certain embodiments, both the primary struts and the secondary struts are made from Nitinol with a transition temperature that is above normal body temperature of humans, which is about 37°C (98.6° F). Thus, when the filter is deployed in the vena cava and exposed to normal body temperature, the struts are in the martensitic state so that the struts are sufficiently ductile to bend or form into a desired shape, which for the present invention is an expanded configuration. To remove the filter, the filter is heated to transform the alloy to austenite so that the filter becomes rigid and returns to a remembered state, which for the filter is a collapsed configuration.

Although the embodiments of this device have been disclosed as being constructed from wire having a round cross section, it could also be cut from a tube of suitable material by laser cutting, electrical discharge machining or any other suitable process.

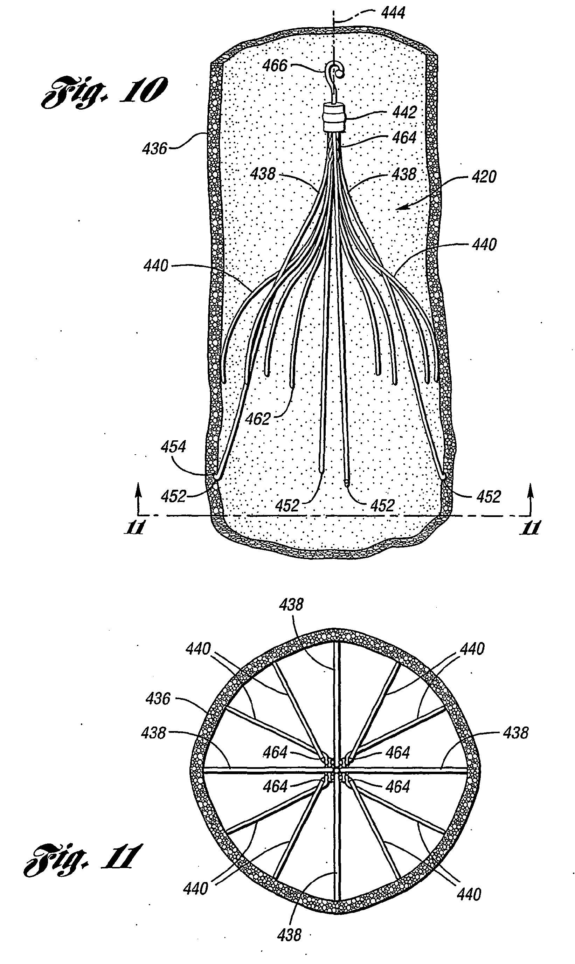

In another embodiment shown in Figures 10 and 11, a filter 420 includes four primary struts 438 and eight secondary struts 440 that extend from a hub 442. Each primary strut 538 terminates in an anchoring hook 452 with a barb 454. The primary struts 438 have sufficient spring strength such that when the filter is deployed in a vena cava 436, the anchoring hooks 452, in particular, the barbs 444, anchor into the vessel wall of the vena cava 436 to prevent the filter 420 from migrating from the delivery location. The pressure of the blood flow on the filter 420 contributes in maintaining the barbs 454 anchored in the inner lining of the vena cava 436.

A pair of secondary struts 440 are positioned between adjacent primary struts 438. Each secondary strut 440 extends from the hub 442 and terminates in a tip 462 pointing toward the central axis 444. The tips 462 are located longitudinally between the hub 442 and the anchoring hooks 454 of the primary struts 438. The connected ends of each pair of secondary struts 440 positioned between adjacent primary struts are twisted together, defining a twisted section 464.

Since the twisted sections 464 effectively stiffens each pair of secondary struts 440, thinner secondary struts may be used to provide the appropriate balancing forces to center the filter in the blood vessel. Moreover, an additional benefit of the twisted section is that they prevent the secondary struts from entangling with the primary struts.

The secondary struts 440 can be made from the same type of material as the primary struts 438 and can be formed by the same process used to form the primary struts. However, the secondary struts may have a smaller diameter than the primary struts. To form the twisted sections 464, each pair of secondary struts 440 positioned between adjacent primary struts 438 can be twisted about each other after the struts have been attached to the hub 442. Each twisted section 464 includes one or more twists. For example, each twisted section 464 may include up to about ten twists. In certain implementations, the number of twists in each section 464 may be between about three to five twists. Increasing the number of twists increases the stiffness of the pair of secondary struts twisted about each other. The hub 442 is preferably made of the same material as the primary struts and secondary struts to minimize the possibility of galvanic corrosion.

Figure 11 illustrates a netting pattern ("net") formed by the primary struts 438, the secondary struts 440, and the hub 442. This net functions to catch thrombi carried in the blood stream to prevent the thrombi from reaching the heart and lungs, where the thrombi could cause pulmonary embolism. The net is sized to catch and stop thrombi that are of a size that are undesirable in the vasculature of the patient. As illustrated, the struts 438 have substantially equal angular spacing between the struts.

The hub 442 and a removal hook 466 attached to the hub are located downstream of the location at which the anchoring hooks 452 are anchored in the vessel 436. When captured by the struts, thrombi remain lodged in the filter 420. The filter 420 along with the thrombi may then be removed percutaneously from the vena cava. When the filter 420 is to be removed, the removal hook 466 is typically grasped by the retrieval hook that is introduced in the vena cava percutaneously.

While the present invention has been described in terms of preferred embodiments, it will be understood, of course, that the invention is not limited thereto since modifications may be made to those skilled in the art, particularly in light of the foregoing teachings.

## Claims

1. A removable filter (10) for capturing thrombi in a blood vessel, the filter comprising: a plurality of primary struts (23) having first ends attached together along a longitudinal axis, each primary strut including an arcuate segment (16) having a first tensile strength and a first thickness, the arcuate segment extending from the first end to an anchoring hook (26), the anchoring hook (26) being integral with the arcuate segment and having the first tensile strength; and a plurality of secondary struts (30), freely spaced between the primary struts and having connected ends attached together along the longitudinal axis, each secondary strut freely extending from the connected end to a free end avoiding contact with other secondary struts and primary struts, **characterised in that** each secondary strut having a second tensile strength which is less than said first tensile strength and a second thickness less thick than the first thickness.

2. The removable filter of claim 1 further comprising: a hub configured to axially house the first ends of the plurality of primary struts; and a retrieval hook (46) extending from the hub opposite the plurality of primary struts for removal of the filter from the blood vessel.

3. The removable filter of claim 1 wherein the first thickness is at least about 0.038 cm (0.015 inch) and the second thickness is at least about 0.030 cm (0.012 inch), and the first absolute tensile strength ranges between1.965 MPa (285,000 psi) and 2.275 MPa (330,000 psi), and the second absolute tensile strength ranges between 1.965 MPa (285,000 psi) and 2.275 MPa (330,000 psi).

4. The filter of claim 1 wherein pairs of secondary struts are positioned between pairs of primary struts, each pair of secondary struts being twisted about each other near the connected ends of the respective secondary struts to form a twisted section.

5. The filter of claim 4 wherein each twisted section includes from one to about ten twists.

6. The removable filter of any one of the preceding claims wherein the arcuate segment includes a first curved portion and a second curved portion, the first curved portion extending from the first end, the second curved portion extending from the first curved portion and terminating at the anchoring hook.

7. The removable filter of claim 6 wherein the first curved portion is configured to extend radially from the central axis of the filter and the second curved portion is configured to extend radially toward the central axis of the filter.

8. The removable filter of claim 6 wherein the first and second curved portions are configured to have a non-parallel relationship with the central axis of the filter.

9. The removable filter of any one of the preceding claims wherein each primary strut is formed of a superelastic material, stainless steel wire, Nitinol, cobalt-chromium-nickel- molybdenum-iron alloy, or cobalt-chrome alloy.

10. The removable filter of any one of the preceding claims wherein each secondary strut is formed of a superelastic material, stainless steel wire, Nitinol, cobalt-chromium-nickel- molybdenum-iron alloy, or cobalt-chrome alloy.

11. The removable filter of any one of the preceding claims wherein the struts are formed of shape memory alloy with a transition temperature.

12. The removable filter of claim 11 wherein the struts expand to the expanded state when the temperature of the struts is about equal to or greater than the transition temperature.

13. The removable filter of claim 11 wherein the struts collapse to the collapsed state when the temperature of struts is about equal to or greater than the transition temperature.

## Patentansprüche

1. Entfernbarer Filter (10) zur Erfassung von Thromben in einem Blutgefäß, wobei der Filter Folgendes umfasst: eine Vielzahl von primären Streben (23) mit ersten Enden, die entlang einer Längsachse aneinander befestigt sind, wobei jede primäre Strebe ein bogenförmiges Segment (16) mit einer ersten Zugfestigkeit und einer ersten Dicke aufweist, wobei sich das bogenförmige Segment vom ersten Ende zu einem Verankerungshaken (26) erstreckt, wobei der Verankerungshaken (26) in das bogenförmige Segment integriert ist und die erste Zugfestigkeit aufweist; und eine Vielzahl von sekundären Streben (30), die mit freiem Abstand zwischen den primären Streben angeordnet sind und verbundene Enden aufweisen, die entlang einer Längsachse aneinander befestigt sind, wobei sich jede sekundäre Strebe frei vom verbundenen Ende bis zu einem freien Ende erstreckt und dabei eine Berührung mit anderen sekundären Streben und primären Streben vermeidet, **dadurch gekennzeichnet, dass** jede sekundäre Strebe eine zweite Zugfestigkeit, die kleiner ist als die erste Zugfestigkeit, und eine zweite Dicke, die weniger dick ist als die erste Dicke, aufweist.

2. Entfernbarer Filter nach Anspruch 1, der ferner Folgendes umfasst: einen Ansatz, so konfiguriert ist, dass er die ersten Enden der Vielzahl von primären Streben beherbergen kann; und einen Rückholhaken (46), der sich vom Ansatz gegenüber der Vielzahl von primären Streben zur Entfernung des Filters aus dem Blutgefäß erstreckt.

3. Entfernbarer Filter nach Anspruch 1, worin die erste Dicke zumindest ungefähr 0,038 cm (0,015 Zoll) und die zweite Dicke mindestens ungefähr 0,030 cm (0,012 Zoll) beträgt und wobei die erste absolute Zugfestigkeit im Bereich von 1,965 MPa (285.000 psi) und 2,275 MPa (330.000 psi) und die zweite absolute Zugfestigkeit im Bereich von 1,965 MPa (285.000 psi) und 2,275 MPa (330.000 psi) liegt.

4. Filter nach Anspruch 1, worin Paare von sekundären Streben zwischen Paaren von primären Streben positioniert sind, wobei jedes Paar von sekundären Streben in der Nähe der verbundenen Enden der jeweiligen sekundären Streben umeinander gewunden sind, um einen verdrehten Abschnitt zu formen.

5. Filter nach Anspruch 4, worin jeder verdrehte Abschnitt ein bis ungefähr 10 Verdrehungen aufweist.

6. Entfernbarer Filter nach einem der vorhergehenden Ansprüche, worin das bogenförmige Segment einen ersten gebogenen Abschnitt und einen zweiten gebogenen Abschnitt aufweist, wobei der erste gebogene Abschnitt sich vom ersten Ende aus erstreckt und der zweite gebogene Abschnitt sich vom ersten gebogenen Abschnitt aus erstreckt und am Verankerungshaken endet.

7. Entfernbarer Filter nach Anspruch 6, worin der erste gebogene Abschnitt so konfiguriert ist, dass er sich radial von der Mittelachse des Filters aus erstreckt, und wobei der zweite gebogene Abschnitt so konfiguriert ist, dass er sich radial zur Mittelachse des Filters erstreckt.

8. Entfernbarer Filter nach Anspruch 6, worin die ersten und zweiten gebogenen Abschnitte so konfiguriert sind, dass sie in nicht-paralleler Beziehung zu der Mittelachse des Filters stehen.

9. Entfernbarer Filter nach einem der vorhergehenden Ansprüche, worin jede primäre Strebe aus einem superelastischen Material, Edelstahldraht, Nitinol, einer Kobalt-Chrom-Nickel-Molybden-EisenLegierung oder einer Kobalt-Chrom-Legierung geformt ist.

10. Entfernbarer Filter nach einem der vorhergehenden Ansprüche, worin jede sekundäre Strebe aus einem superelastischen Material, Edelstahldraht, Nitinol, einer Kobalt-Chrom-Nickel-Molybdän-EisenLegierung oder einer Kobalt-Chrom-Legierung geformt ist.

11. Entfernbarer Filter nach einem der vorhergehenden Ansprüche, worin die Streben aus einer Formgedächtnislegierung mit einer Übergangstemperatur geformt sind.

12. Entfernbarer Filter nach Anspruch 11, worin die Streben zu dem expandierten Zustand expandieren, wenn die Temperatur der Streben ungefähr gleich oder größer als die Übergangstemperatur ist.

13. Entfernbarer Filter nach Anspruch 11, worin die Streben in den kollabierten Zustand kollabieren, wenn die Temperatur der Streben ungefähr gleich oder größer als die Übergangstemperatur ist.

## Revendications

1. Filtre (10) retirable pour recueillir les caillots thrombolytiques dans un vaisseau sanguin, le filtre comportant : une pluralité d'entretoises (23) primaires présentant plusieurs premières extrémités attachées ensemble le long d'un axe longitudinal, chaque entretoise primaire comprenant un segment arqué (16) possédant une première résistance à la traction et une première épaisseur, le segment arqué s'étendant depuis la première extrémité jusqu'à un crochet (26) d'ancrage, le crochet (26) d'ancrage étant d'un seul tenant avec le segment arqué et ayant la première résistance à la traction ; et une pluralité d'entretoises (30) secondaires espacées librement entre les entretoises primaires et présentant des extrémités reliées attachées ensemble le long de l'axe longitudinal, chaque entretoise secondaire s'étendant librement depuis l'extrémité reliée à une extrémité libre évitant tout contact avec d'autres entretoises secondaires et entretoises primaires, **caractérisé en ce que** chaque entretoise secondaire a une deuxième résistance à la traction qui est inférieure à ladite première résistance à la traction et une deuxième épaisseur moins élevée que la première épaisseur.

2. Filtre retirable selon la revendication 1 comportant en outre : un moyeu conçu pour loger axialement les premières extrémités de la pluralité d'entretoises primaires ; et un crochet (46) de récupération s'étendant depuis le moyeu, à l'opposé de la pluralité d'entretoises primaires pour l'enlèvement du filtre du vaisseau sanguin.

3. Filtre retirable selon la revendication 1 dans lequel la première épaisseur est au moins de l'ordre d'environ 0,038 cm (0,015 pouces) et la deuxième épaisseur est au moins de l'ordre d'environ 0,030 cm (0,012 pouces), et la première résistance à la traction absolue se situe entre 1,965 MPa (285 000 psi) et 2,275 MPa (330 000 psi), et la deuxième résistance à la traction absolue se situe entre 1,965 MPa (285 000 psi) et 2,275 MPa (330 000 psi).

4. Filtre selon la revendication 1 dans lequel des paires d'entretoises secondaires sont positionnées entre des paires d'entretoises primaires, chaque paire d'entretoises secondaires étant torsadée les unes autour des autres près des extrémités reliées des entretoises secondaires respectives pour former une section torsadée.

5. Filtre selon la revendication 4 dans lequel chaque section torsadée comprend de une à dix torsions environ.

6. Filtre retirable selon l'une quelconque des revendications précédentes dans lequel le segment arqué comprend une première portion courbe et une deuxième portion courbe, la première portion courbe s'étendant depuis la première extrémité, la deuxième portion courbe s'étendant depuis la première portion courbe et se terminant au niveau du crochet d'ancrage.

7. Filtre retirable selon la revendication 6 dans lequel la première portion courbe est conçue pour s'étendre radialement depuis l'axe central du filtre et la deuxième portion courbe est conçue pour s'étendre radialement vers l'axe central du filtre.

8. Filtre retirable selon la revendication 6 dans lequel les première et deuxième portions courbes sont conçues pour avoir une relation non parallèle avec l'axe central du filtre.

9. Filtre retirable selon l'une quelconque des revendications précédentes dans lequel chaque entretoise primaire est formée dans un matériau superélastique, du fil en acier inoxydable, du nitinol, un alliage cobalt-chrome-molybdène-nickel-fer, ou un alliage cobalt-chrome.

10. Filtre retirable selon l'une quelconque des revendications précédentes dans lequel chaque entretoise est réalisée dans un matériau superélastique, du fil en acier inoxydable, du nitinol, un alliage cobalt-chrome-molybdène-nickel-fer, ou un alliage cobalt-chrome.

11. Filtre retirable selon l'une quelconque des revendications précédentes dans lequel les entretoises sont formées d'un alliage à mémoire de forme possédant une température de transition.

12. Filtre retirable selon la revendication 11 dans lequel les entretoises se déploient vers un état déployé lorsque la température des entretoises est à peu près égale ou supérieure à la température de transition.

13. Filtre retirable selon la revendication 11 dans lequel les entretoises se replient vers un état replié lorsque la température des entretoises est environ égale ou supérieure à la température de transition.
